Europäisches Patentamt

European Patent Office

Office européen des brevets

(11) Publication number: **0 187 433 B1**

(12) **EUROPEAN PATENT SPECIFICATION**

(45) Date of publication of patent specification: **27.12.90**

(21) Application number: **85300205.3**

(22) Date of filing: **11.01.85**

(51) Int. Cl.⁵: **A 61 K 9/06, A 61 K 47/00, A 61 K 47/12, A 61 K 47/38**

(54) **Powdery pharmaceutical composition suitable for application to mucosa of oral or nasal cavity.**

(43) Date of publication of application:
**16.07.86 Bulletin 86/29**

(45) Publication of the grant of the patent:
**27.12.90 Bulletin 90/52**

(84) Designated Contracting States:
**CH DE FR GB IT LI NL**

(56) References cited:
**EP-A-0 023 359**
**US-A-3 276 959**
**US-A-4 289 764**

(73) Proprietor: **TEIJIN LIMITED**
**11 Minami Honmachi 1-chome Higashi-ku**
**Osaka-shi Osaka-fu (JP)**

(72) Inventor: **Suzuki, Yoshiki**
**5-20-2, Tamadaira**
**Hino-shi Tokyo (JP)**
Inventor: **Ikura, Hiroshi**
**Gurin-kopu Hino 2-501,2-14-1**
**Shinmei Hino-shi Tokyo (JP)**

(74) Representative: **Arthur, Bryan Edward et al**
**Withers & Rogers 4 Dyer's Buildings Holborn**
**London EC1N 2JT (GB)**

Courier Press, Leamington Spa, England.

## EP 0 187 433 B1

**Description**

The present invention relates to a powdery pharmaceutical composition suitable for application to the mucosa of the oral or nasal cavity. More specifically, it relates to a powdery pharmaceutical composition, suitable for application to the mucosa of the oral or nasal cavity, containing (a) lower alkyl ethers of cellulose as a base, (b) a steroid or glycyrrhizic acid type anti-inflammatory agent as a drug component, and (c) a solid low irritant organic acid as a stabilizer for the drug component.

The powdery pharmaceutical composition exhibits extremely excellent stability in the steroid or glycyrrhizic acid type anti-inflammatory agent contained therein, low irritation of the mucosa of the oral or nasal cavity, and sustained release of the drug over an extended period of time. Accordingly, the powdery pharmaceutical composition according to the present invention can be effectively used for the treatment of oral diseases such as stomatitis, erosion or sores generated by radiotherapy, and licken planus or nasal diseases such as allergic rhinitis and vasomotor rhinitis.

Various pharmaceutical preparations, such as buccal tablets, troche tables, sublingual tablets, and oral ointments, have been heretofore known in the art for application to the oral cavity. Buccal tablets, troche tablets, and sublingual tablets, however, give a foreign feeling to the oral cavity. Therefore, patients often chew on and swallow the tables. Oral ointments on the other hand, usually contain mixtures of beeswax or plastibase with gelatin, pectin, or sodim carboxymethyl cellulose. These mixtures are unsatisfactory because the adhesion properties thereof to the oral mucosa are not sufficient and also because they have unpleasant tastes.

On the other hand, various pharmaceutical preparations, such as nasal ointments, jellies, nose drops, and sprays, have been known in the art for application to the nasal cavity. Nasal ointments and jellies, however, are difficult to apply to deep parts of the nasal cavity, such as the concha nasalis superior. With nose drops and sprays, the active components do not remain in the nasal cavity for an extended period of time.

Various sustained release pharmaceutical preparations for oral or nasal cavity administration are known in the art. With these, the efficacy of the drugs is spread over a long period of time by the gradual or sustained releasing the active drug components from the pharmaceutical preparations. For example, Japanese Unexamined Patent Publication (Kokai) No. 57-118511 discloses powdery sustained release preparations suitable for adhesion to the oral cavity. These preparations use, as a base, hydroxypropyl cellulose and have the advantages of an excellent sustained release of the drugs and possible application over a wide range of the oral cavity. Furthermore, U.S. Patent No. 4,294,829 discloses powdery sustained release preparations suitable for application to the nasal cavity. These contain lower alkyl ethers of cellulose and drugs and also have the advantage of excellent sustained release of the drugs.

It is, however, more desirable to improve the stability of drugs in the above-mentioned sustained release preparations for application to the mucosa of the oral or nasal cavity.

Accordingly, the object of the present invention is to provide a powdery pharmaceutical composition, suitable for application to the mucusa of the oral or nasal cavity, having extremely excellent stability of the carried drug, i.e., a steroid or glycyrrhizic acid type anti-inflammatory agent.

Another object of the present invention is to provide a powdery pharmaceutical composition, suitable for application to the mucosa of the oral or nasal cavity, containing a lower alkyl ether or cellulose as a base, a steroid of glycyrrhizic acid type anti-inflammatory agent as a drug, and a specific solid low irritant organic acid as a stabilizer for the drug.

A further object of the present invention is to provide a powdery pharmaceutical composition having low irritation of the mucosa of the oral or nasal cavity.

A still further object of the present invention is to provide a powdery pharmaceutical composition having excellent sustained release of the drug.

A still further object of the present invention is to provide a powdery pharmaceutical composition having a particle diameter suitable for administration to the oral or nasal cavity.

A still further object of the present invention is to provide a method for preparing a powdery pharmaceutical composition suitable for application to the mucosa of the oral or nasal cavity.

A still further object of the present invention is to provide a method for stabilizing a powdery pharmaceutical composition containing a steroid or glycyrrhizic acid type anti-inflammatory agent as a drug.

Other objects and advantages of the present invention will be apparent from the following description.

According to the present invention, a powdery pharmaceutical composition for application to the mucosa of the oral or nasal cavity comprises a base selected from lower alkyl ethers or cellulose, a pharmaceutically effective amount of a drug selected from steroidal anti-inflammatory agents or glycyrrhizic acid anti-inflammatory agents and, as a solid stabilizer for the drug, at least one low irritant solid organic acid selected from saturated higher aliphatic monocarboxylic acids having 12 tm 24 carbon atoms, aliphatic hydrocarbon dicarboxylic acids having 4 to 6 carbon atoms, hydroxy aliphatic hydrocarbon di-carboxylic and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition.

2

EP 0 187 433 B1

The lower alkyl ethers of cellulose usable as a base in the present invention are those obtained by at least partially substituting the same or different lower alkyl ethers groups for a plurality of hydroxyl groups of cellulose. The lower alkyl gropus in the lower alkyl ether groups may be substituted with substituents. An example of such preferable substituents is a hydroxyl group.

Preferable examples of the optionally substituted lower alkyl groups are a methyl group, and hydroxy lower alkyl groups having 2 or 3 carbon atoms.

Examples of the optionally substituted lower alkyl groups are methyl, ethyl, n-propyl, iso-propyl, β-hydroxyethyl, and β-hydroxypropyl.

Examples of the optionally substituted lower alkyl ethers of cellulose are methyl cellulose, hydroxyethyl cellulose, hydroxypropyl cellulose, and hydroxypropylmethyl cellulose. Of these lower alkyl ethers of cellulose, the use of methyl cellulose, hydroxypropyl cellulose, and hydroxypropylmethyl cellulose is preferable due to the facts that these lower alkyl ethers of cellulose have substantially no odor and irritation when applied to the mucosa of the oral or nasal cavity, which is particularly sensitive to odor and irritation, and that the desired sustained release of the drug can be obtained. Of these lower alkyl ethers of cellulose, the use of hydroxypropyl cellulose is especially preferable.

The lower alkyl ethers of cellulose can be used alone or in any mixture thereof.

When the above-mentioned lower alkyl ethers of cellulose are used as a base in the powdery pharmaceutical composition according to the present invention, the pharmaceutical preparations thus obtained can adhere to the mucosa of the oral or nasal cavity and will swell to gradually release the active drug components therefrom over a long period of time. Thus, the affected or diseased parts can be treated by the drugs over a long period of time.

Although there is no limitation in the molecular weight of the lower alkyl ethers of cellulose usable in the present invention, lower alkyl ethers having a viscosity in 2% aqueous solution thereof of 3 to 10,000 mPas (cps) at 37°C±0.2°C, more preferably, 1000 to 4000 mPas (cps) at 37°C±0.2°C, are preferably used.

In preparing unit dosage forms of powdery pharmaceutical compositions in which lower alkyl ethers of cellulose are contained as a base, the compositions are generally filled in hard capsules. These capsules are set in a specially designed spraying device. The spraying device is provided with needle for spraying the powdery pharmaceutical compositions into the oral or nasal cavity. In this case, when the moisture content of the lower alkyl ethers of cellulose in the capsules is too small, the moisture contained in the membranes of the capsules tends to be absorbed by the lower alkyl ethes of cellulose and, as a result, the capsules become too rigid. Therefore, lower alkyl ethers of cellulose having a moisture content of 3% to 9% by weight, especially 4% to 8% by weight, are preferably used as a base.

The drugs usable in the present powdery pharmaceutical compositions are steroid or glycyrrhizic acid type anti-inflammatory agents. Examples of such steroid type anti-inflammatory agents are beclomethasone dipropionate, betamethasone, betamethasone valerate, triamcinolone, triamcinolone acetonide, dexamethasone, fluocinolone acetonide, fluocinonide, flumethasone, hydrocortisone, prednisolone, and prednisone. Examples of glycyrrhizic acid type anti-inflammatory agents are glycyrrhizic acid, disodium glycyrrhizinate, trisodium glycyrrhizinate, monopotassium glycyrrhizinate, dipotassium glycyrrhizinate and monoammonium glycyrrhizinate. These drugs can be used alone or in any mixture thereof in the present powdery pharmaceutical composition.

According to the present invention, the above-mentioned anti-inflammatory agents contained in the powdery pharmaceutical compositions comprising the above-mentioned lower alkyl ethers of cellulose as a base can be advantageously stabilized by incorporating thereinto, as a stabilizer, a specified organic acid, i.e., at least one solid low irritant organic acid selected from saturated higher alihpatic monocarboxylic acids having 12 or more carbon atoms, aliphatic polycarboxylic acids, hydroxy aliphatic polycarboxylic acids, aromatic carboxylic acids, unsaturated lower aliphatic monocarboxylic acids having six or less carbon atoms, and cellulose derivatives having carboxyl groups.

The organic acids usable as a stabilizer for the drug in the present invention are those selected from the above-mentioned group of the organic acids which are solid at room temperature, less irritant to the mucosa of the oral or nasal cavity, and substantially odorless or with only a very slight odor. By the term "organic acids" is meant acids comprising organic groups and free carboxylic groups. Use of the organic acids solid at room temperature facilitates the production of the desired powder pharmaceutical compositions in a uniform particle size. Use of the less irritant organic acids means higher safety when the composition is applied to the mucosa of the oral or nasal cavity.

Examples of the preferable saturated higher aliphatic monocarboxylic acids are saturated higher aliphatic hydrocarbon monocarboxylic acid having 12 to 24 carbon atoms, such as stearic acid, palmitic acid, lauric acid, and myristic acid. Examples of the preferable aliphatic polycarboxlic acids are aliphatic hydrocarbon dicarboxylic acids having 4 to 6 carbon atoms, such as, succinic acid, fumaric acid, and maleic acid. Examples of the preferable hydroxy aliphatic polycarboxylic acid are hydroxy aliphatic hydrocarbon di- or tri-carboxylic acids having 4 to 8 carbon atoms, such as, tartaric acid and citric acid. Examples of the preferably aromatic acids are benzoic acid and its derivatives, such as parahydroxy benzoic acid and salicylic acid. Examples of the preferable unsaturated lower aliphatic monocarboxylic acids are unsaturated lower aliphatic hydrocarbon monocarboxylic acid having 4 to 6 carbon atoms, such as sorbic acid. Examples of the preferable cellulose derivatives having carboxyl groups are carboxymethyl cellulose and carboxymethylethyl cellulose. These organic acids can be used alone or in any mixture thereof.

3

Of the above-mentioned organic acids, saturated higher aliphatic monocarboxylic acids having 12 or more carbon atoms, aliphatic polycarboxylic acids, hydroxy aliphatic polycarboxylic acids, aromatic carboxylic acids, and cellulose derivatives having carboxyl groups are preferably used. For powdery pharmaceutical compositions for application to the nasal cavity, use of saturated higher aliphatic monocarboxylic acids having 12 or more carbon atoms and cellulose derivatives having carboxyl groups is especially preferably from the view-points of low irritation and substantial odorlessness.

According to a preferred feature of the invention, the amount of the organic acid in the present pharmaceutical composition is within the range of from 0.1% to 3% by weight, based upon the total weight of the composition. Although there is no critical limitation in the particle size of the organic acid, the organic acid preferably has such a particle size that at least about 90% by weight of the powder particles has an effective particle diameter of about 75 µm, or less, more preferably about 20 µm to about 75 µm, to obtain a powdery pharmaceutical composition capable of being effectively administered to the mucosa of the oral or nasal cavity.

As mentioned above, the powdery pharmaceutical compositions according to the present invention contain, as essential constituents, the above-mentioned lower alkyl ethers of cellulose, steroid or glycyrrhizic acid type anti-inflammatory agents, and organic acid stabilizers. Although there is no specific limitation in the particle size of the present pharmaceutical composition, the particle size of the present pharmaceutical composition is preferably such that at least about 90% by weight of the powder particles thereof has an effective particle diameter of about 20 µm to about 250 µm. The present powdery pharmaceutical composition having such a particle size distribution is preferably due to the fact that the amount of the powder particles adhering to the mucosa of the oral or nasal cavity becomes large. That is, when the pharmaceutical composition having the above-mentioned preferable particle size distribution is applied to the oral or nasal cavity, the amount of the pharmaceutical composition reaching, for example, the lung becomes small or the amount of the pharmaceutical composition scattered out of the oral or nasal cavity becomes small. Furthermore, the pharmaceutical composition having the above-mentioned preferable particle size can well adhere to the mucosa of the oral or nasal cavity and can be effectively and gradually released. The powdery pharmacetical composition according to the present invention more preferably has such a particle size distribution that at least about 90% by weight of the powder particles has an effective particle diameter of about 20 µm to about 150 µm.

The term "effective particle diameter" used herein means that determined by the opening sizes of sieves. For example, a powder having an effective diameter (d) of $37 < d \leqq 44$ passes through a sieve having an opening size of 44 µm but does not pass through a sieve having an opening size of 37 µm.

A vibratory sieve is used when the effective particle diameter of a powder is more than 37 µm, and a sonic sieve (Micro Hand Sifter SWM-2, a product of Tsutsui Rikagaku Kikai Co., Ltd.) is used when the effective particle diameter of a powder is not more than 37 µm.

The sieves used has an opening size of 500, 420, 350, 297, 250, 210, 177, 149, 125, 105, 88, 74, 63, 53, 44, 37, 25, and 20 µm, respectively.

The powdery pharmaceutical composition according to the present invention can optionally contain, in addition to the above mentioned lower alkyl ethers of cellulose, anti-inflammatory agents, and organic acids, any conventiontional ingredients for improving the physical properties, visual appearance, or odor of the pharmaceutical preparations. Examples of such ingredients are: lubricants such as talc and waxes; binders such as starch, dextrin, tragacanth gum, gelatin, polyvinylpyrrolidone, and polyvinyl alcohol; diluents such as starch, crystalline cellulose, dextrin, lactose, mannitol, sorbitol, and anhyhdrous calcium phosphate; odor improvers such as menthol and citrus perfumes; preservatives such as methyl p-hydroxybenzoate, propyl p-hydroxybenzoate; and various surfactants.

The powdery pharmaceutical composition according to the present invention can be prepared in any conventional manner. For example, the pharmaceutical composition can be prepared by mechanically mixing the above-mentioned three essential constituents, i.e., the lower alkyl ethers of cellulose, the anti-inflammatory agents, and the solid organic acids and the above-mentioned optional ingredients, if any. The mixing of the composition can be carried out by using any conventional means such as ball mills, and V-type mixers. The desired pharmaceutical composition can also be prepared by mechanically mixing all the above-mentioned constituents of the composition, compressing the resultant mixture under pressure, e.g., 0.2 to 0.4 ton, to and pulverizing the compressed mixture so as to obtain the desired particle size distribution. The mixed or pulverized powder particles can be sieved to obtain the powdery composition having the desired particle size distribution, if necessary.

The desired pharmaceutical composition can further be prepared by impregnating the lower alkyl ethers of cellulose with a solution or dispersion, in an organic solvent (e.g., methanol, ethanol, ethyl cellosolve, and dichloromethane), of the anti-inflammatory agents and the organic acids and, if any, the optional ingredients, followed by drying the impregnated product. The said organic solvent may contain water. The impregnation can be carried out by, for example, spraying the above-mentioned solution or dispersion to the lower alkyl ethers of cellulose in the form of powder particles in a spray apparatus. The particle size distribution of the lower alkyl ethers of cellulose can be controlled to the desired range prior to the impregnation. Alternatively, the powder particles of the impregnated product can be pulverized and/or sieved after the impregnation so as to obtain the desired particle size distribution.

Furthermore, the desired pharmaceutical compositions can be prepared by dissolving or dispersing

4

# EP 0 187 433 B1

the lower alkyl ethers of cellulose, the anti-inflammatory agents, the solid organic acid, and any optional ingredients in organic solvents such as methanol, ethanol, ethyl cellosolve, and dichloromethane, water, or the mixture thereof followed by evaporating the organic solvents off. The resultant composition can be pulverized and/or sieved to obtain the desired particle size distribution.

According to the present invention, a powdery preparation in a unit dosage form including the above-mentioned powdery pharmaceutical composition can be obtained. The present pharmaceutical composition can be directly converted to the powdery preparation in the form of a unit dosage. The unit dosage of the powdery preparations according to the present invention is preferably 10 mg to 400 mg, especially 30 mg to 300 mg, when applied to the oral cavity and 5 mg to 200 mg, especially 10 mg to 100 mg, when applied to the nasal cavity.

The amount of the steroid or glycyrrhizic acid type anti-inflammatory agent in the powdery preparation in the unit dosage form depends upon the pharmaceutically effective amount of the anti-inflammatory agent to be used and the number of dosages of the preparation. For example, when beclomethasome dipropionate is incorporated into the powdery preparations for application to the nasal or oral cavity, the appropriate amount is about 25 to 200 µg/day, although the conventional amount is about 200 to 400 µg/day. When dipotassium glycyrrhizinate is incorporated into the powdery preparations for application to the oral cavity, the appropriate amount is about 0.1 to 15 mg/day, although the conventional amount is about 10 to 30 mg/day. This is because, since lower alkyl ethers of cellulose are used in the present pharmaceutical composition, a desired sustained release of the drug component can be obtained.

The powdery preparations of the present invention can be preferably filled into capsules, such as hard gelatin capsules, as a preferred dosage form.

The present powdery preparations can be advantageously applied to the oral or nasal cavity. For exampkle, a capsule filled with the present powdery preparation is set in a spraying device equipped with a needle. The capsule is pierced with the needle to provide minute holes on the top and bottom sides. The powdery preparation is then sprayed or jetted out from the capsule by sending air into the capsule by means of, for example, a rubber ball.

As mentioned above, when the moisture content in the powdery preparation filled in a hard gelatin capsule is too small, the powdery preparation absorbs moisture from the gelatine membrane. Therefore, water is preferably contained in an amount of 3% to 8.5% by weight, especially 4% to 8.5% by weight, in the lower alkyl ethers of cellulose in the powdery preparations.

The powdery preparations according to the present invention can be widely used for the treatment of oral diseases such as stomatitis, erosion or sores generated by radiotherapy, and lichen planus or nasal diseases such as allergic rhinitis and vasomotor rhinitis.

## Example

The present invention will now be described in detail with reference to, but is by no means limited to, the following examples, wherein all percentages and parts are on the weight basis unless otherwise specified.

## Example 1

A 100 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 µm was used.

One part of the hydroxypropyl cellulose and 0.025 parts of beclomethasone dipropionate were thoroughly mixed in a mortar and, then, 9 parts of hydroxypropyl cellulose was gradually added thereto. The resultant mixture was thoroughly mixed and, then, the remaining hydroxypropyl cellulose were added and thoroughly mixed in a mixer. Thereafter, 0.5 parts of stearic acid was added and thoroughly mixed.

Thus, a powdery composition for the application to the oral cavity containing beclomethasome dipropionate uniformly dispersed therein was prepared. The powdery composition was filled in #2 hard gelatin capsules in an amount of 200 mg each to prepare a powdery preparation in a unit dosage form for the application to the oral cavity.

## Example 2

A powdery preparation in a unit dosage form for the application to the oral cavity was prepared in the same manner as in Example 1, except that palmitic acid was used instead of stearic acid.

## Example 3

A powdery preparation in a unit dosage form for the application to the oral cavity was prepared in the same manner as in Example 1, except that citric acid was used instead of stearic acid.

## Example 4

A powdery preparation in a unit dosage form for the application to the oral cavity was prepared in the same manner as in Example 1, except that benzoic acid was used instead of stearic acid.

Example 5

A powdery preparation in a unit dosage form for the application to the oral cavity was prepared in the same manner as in Example 1, except that sorbic acid was used instead of stearic acid.

Example 6

Powdery compositions for the application to the oral cavity were prepared in the same manner as in Example 1, except that the amounts of stearic acid were changed to 0, 0.1, 0.3, 0.5, and 0.7 parts. These powdery compositions were filled in #2 hard gelatin capsules in an amount of 200 mg each.

The capsules thus prepared were allowed to stand at a temperature of 60°C for one month. The residual percentages of beclomethasone dipropionate in the capsules were determined.

The results were as shown in Table 1.

TABLE 1

| Content of stearic acid (%) | 0 | 0.1 | 0.3 | 0.5 | 0.7 |
|---|---|---|---|---|---|
| Residual percentage of beclo-methasone dipropionate (%) | 75.0 | 89.0 | 95.2 | 97.8 | 99.2 |

Example 7

Powdery compositions for the application to the oral cavity were prepared in the same manner as in Example 2, except that the amounts of palmitic acid were changed to 0, 0.1, 0.3, and 0.5 parts. These powdery compositions were filled in #2 hard gelatin capcules in an amount of 200 mg each.

The capsules thus prepared were allowed to stand at a temperature of 60°C for one month. The residual percentages of beclomethasone dipropionate in the capsules were determined.

The results were as shown in Table 2.

TABLE 2

| Content of palmitic acid (%) | 0 | 0.1 | 0.3 | 0.5 |
|---|---|---|---|---|
| Residual percentage of beclo-methasone dipropionate (%) | 78.5 | 90.0 | 95.2 | 98.1 |

Example 8

Powdery compositions for the application to the oral cavity were prepared in the same manner as in Example 3, except that the amounts of citric acid were changed to 0, 0.05, 0.1 0,5, 1, and 3 parts. These powdery compositions were filled in #2 hard gelatin capsules in an amount of 200 mg each.

The capsules thus prepared were allowed to stand at a temperature of 60°C for one month. The residual percentages of beclomethasone dipropionate in the capsules were determined.

The results were as shown in Table 3.

TABLE 3

| Content of citric acid (%) | 0 | 0.05 | 0.1 | 0.5 | 1 | 3 |
|---|---|---|---|---|---|---|
| Residual percentage of beclo-methasone dipropionate (%) | 78.5 | 92.0 | 94.3 | 100.0 | 100.0 | 100.0 |

Example 9

Powdery compositions for the application to the oral cavity were prepared in the same manner as in Example 4, except that the amounts of benzoic acid were changed to 0, 0.05, 0.1, and 0.5 parts. These powdery compositions were filled in #2 hard gelatin capsules in an amount of 200 mg each.

The capsules thus prepared were allowed to stand at a temperature of 60°C for one month. The residual percentages of beclomethasone dipropionate in the capsules were determined.

The results were as shown in Table 4.

TABLE 4

| Content of benzoic acid (%) | 0 | 0.05 | 0.1 | 0.5 |
|---|---|---|---|---|
| Residual percentage of beclomethasone dipropionate (%) | 82.5 | 93.2 | 94.0 | 100.0 |

Example 10

Powdery compositions for the application to the oral cavity were prepared in the same manner as in Example 5, except that the amounts of sorbic acid were changed to 0, 0.1, 0.3, and 0.5 parts. These powdery compositions were filled in #2 hard gelatin capsules in an amount of 200 mg each.

The capsules thus prepared were allowed to stand at a temperature of 60°C for one month. The residual percentages of beclomethasone dipropionate in the capsules were determined.

The results were as shown in Table 5.

TABLE 5

| Content of sorbic acid (%) | 0 | 0.1 | 0.3 | 0.5 |
|---|---|---|---|---|
| Residual percentage of beclomethasone diproprionate (%) | 83.1 | 92.6 | 98.2 | 98.9 |

Exampe 11

Powdery preparations for the application to the oral cavity were prepared in the same manner, except that 100 parts of methyl cellulose having a viscosity of a 2% aqueous solution thereof of 1335 mPas (cps) at 37°C±0.2°C and 0.025 parts of triamcinolon acetonide were used and that the amounts of the stearic acid were changed to 0, 0.1, 0.3, and 0.5 parts.

The residual percentages of the triamcinolon acetonide were determined after the capsuled preparations were allowed to stand at a temperature of 60°C for one month.

The results were as shown in Table 6.

TABLE 6

| Content of stearic acid (%) | 0 | 0.1 | 0.3 | 0.5 |
|---|---|---|---|---|
| Residual percentage of triamcinolone acetonide (%) | 81.0 | 92.6 | 97.8 | 100.0 |

Example 12

A 100 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 μm was used.

A 10 part amount part of the hydroxypropyl cellulose and 0.17 parts of beclomethasone dipropionate were thoroughly mixed in a mixer and, then, the remaining hydroxypropyl cellulose was gradually added thereto. The resultant mixture was thoroughly mixed. Thereafter, 0.5 parts of stearic acid was added and thoroughly mixed.

Thus, a powdery composition for the application to the nasal cavity containing beclomethasone dipropionate uniformly dispersed therein was prepared. The powdery composition was filled in #2 hard gelatin capsules in an amount of 30 mg each to prepare a powdery preparation in a unit dosage form for the application to the nasal cavity.

Example 13

A 100 part amount of methyl cellulose having a viscosity of a 2% aqueous solution thereof of 1335 mPas (cps) at 37°C±0.2°C was used.

A 10 part amount of the methyl cellulose and 0.17 parts of beclomethasone dipropionate were thoroughly mixed in a mortar and, then, the remaining hydroxypropyl cellulose was gradually added thereto. The resultant mixture was thoroughly mixed. Thereafter, 0.5 parts of stearic acid was added and thoroughly mixed.

7

Thus, a powdery composition for the application to the nasal cavity containing beclomethasone dipropionate uniformly dispersed therein was prepared. The powdery composition was filled in #2 hard gelatin capsules in an amount of 30 mg each to prepare a powdery preparation in a unit dosage form for the application to the nasal cavity.

Example 14

A powder preparation in a unit dosage form for the application to the nasal cavity was prepared in the same manner as in Example 13, except that hydroxypropylmethyl cellulose having a viscosity of a 2% aqueous solution thereof of 20 mPas (cps) at 37°C±0.2°C was used instead of the methyl cellulose. The powdery composition was filled in a #2 hard gelatin capsules in an amount of 30 mg each to prepare a powdery preparation in a unit dosage form for the application to the nasal cavity.

Example 15

Powdery compositions were prepared in the same manner s in Example 12, except that the amounts of stearic acid were changed to 0, 0.01, 0.1, 0.3, 0.5, and 0.7 parts. These powdery compositions were filled in #2 hard gelatin capsules in an amount of 30 mg each.

The capsules thus prepared were allowed to stand at a temperature of 60°C for one month. The residual percentages of beclomethasone dipropionate in the capsules were determined.

The results were as shown in Table 7.

TABLE 7

| Content of stearic acid (%) | 0 | 0.1 | 0.3 | 0.5 | 0.7 |
|---|---|---|---|---|---|
| Residual percentage of beclo-methasone dipropionate (%) | 77.5 | 87.0 | 93.3 | 96.7 | 98.3 |

Example 16

The same evaluation test of Example 15 was carried out, except that palmitic acid was used instead of the stearic acid.

The result were as shown in Table 8.

TABLE 8

| Content of palmitic acid (%) | 0 | 0.1 | 0.3 | 0.5 | 0.7 |
|---|---|---|---|---|---|
| Residual percentage of beclo-methasone dipropionate (%) | 78.5 | 92.0 | 93.8 | 96.2 | 98.1 |

Example 17

Powdery compositions were prepared in the same manner as in Example 12, except that the carboxymethyl cellulose was used, instead of the stearic acid in amounts of 0, 1, 2, and 3 parts. These powdery compositions were filled in #2 hard gelatin capsules in an amount of 30 mg each.

The capsules thus prepared were allowed to stand at a temperature of 60°C for one month. The residual percentages of beclomethasone dipropionate in the capsules were determined.

The results were as shown in Table 9.

TABLE 9

| Content of carboxymethyl cellulose (%) | 0 | 1 | 2 | 3 |
|---|---|---|---|---|
| Residual percentage of beclo-methasone dipropionate (%) | 82.6 | 92.1 | 93.2 | 95.8 |

Example 18

Powdery compositions were prepared in the same manner as in Example 13, except that the amounts of stearic acid were changed to 0, 0.01, 0.05, 0.1, and 0.5 parts. These powdery compositions were filled in #2 hard gelatin capsules in an amount of 30 mg each.

The capsules thus prepared were allowed to stand at a temperature of 60°C for one month. The residual percentages of beclomethasone dipropionate in the capsules were determined.
The results were as shown in Table 10.

TABLE 10

| Content of stearic acid (%) | 0 | 0.05 | 0.1 | 0.5 |
|---|---|---|---|---|
| Residual percentage of beclo-methasone dipropionate (%) | 75.5 | 90.2 | 92.3 | 96.8 |

Example 19

A 90 part amount of hydroxypropyl cellulose having a viscosity of a 2% aqueous solution of 1550 mPas (cps) at 37°C±0.2°C and having a particle size such that 90% or more of the particles thereof had a particle diameter of 37 to 149 µm and 10 parts of dipotassium glycyrrhizinate were thoroughly mixed in a mixer. Then, 0.1, 0.3, 0.5, and 0.7 part of stearic acid were added to the resultant mixture to prepare powdery compositions for the application to the oral cavity.

The powdery compkositions prepared above were filled into #2 gelatin hard capsules in an amount of 100 mg each. The capsules thus obtained were allowed to stand at a temperature of 60°C for one month. The residual percentages of the glycyrrhizinate in the capsules were determined.

The results were as shown in Table 11.

TABLE 11

| Content of stearic acid (%) | 0 | 0.5 | 0.7 |
|---|---|---|---|
| Residual percentage of glycyrrhizinate (%) | 92.0 | 100 | 100 |

Example 20

A 100 part amount of hydroxypropyl cellulose having a viscosity of 2% aqueous solution thereof of 1550 mPas (cps) at 37°C±0.2°C and having particle size such that 90% or more of the powder particles had a particle of 37 to 149 µm was used.

One part of the hydroxypropyl cellulose and 0.025 part of beclomethasone dipropionate were thoroughly mixed in a mortar and, then, 9 parts of hydroxypropyl cellulose were gradually added thereto. The resultant mixture was thoroughly mixed and, then, the remaining hydroxypropyl cellulose was added and thoroughly mixed in a mixer. Thereafter, 0.5 part of stearic acid was added and thoroughly mixed.

Thus, a powdery comvosition for the application to the oral cavity containing beclomethasone dipropionate uniformly dispersed therein was prepared. The powdery composition was filled in #2 hard gelatin capsules in an amount of 200 mg each to prepare a powdery preparation in a unit dosage form for the application to the oral cavity.

The capsules thus prepared were allowed to stand 60°C for one month and at 40°C for 6 months.

TABLE 12

| | Storage period (day) | 7 | 14 | 21 | 30 |
|---|---|---|---|---|---|
| 60°C | Residual percentage of beclomethasone dipropionate (%) | 100.0 | 99.8 | 98.0 | 97.8 |
| | Storage period (month) | 1 | 2 | 3 | 6 |
| 40°C | Residual percentage of beclomethasone dipropionate (%) | 100.0 | 99.0 | 98.7 | 97.5 |

Comparative Example 1

A 100 part amount of hydropropyl cellulose having a viscosity of 2% aqueous solution thereof of 1550 mPas (cps) at 37°C±0.2°C and having a particle size such that 90% or more of the powder particles had a particle diameter of 37 to 149 µm was used.

One part of the hydroxypropyl cellulose and 0.025 part of beclomethasone dipropionate were thoroughly mixed in a mortar and, then, 9 parts of hydroxypropyl cellulose was gradually added thereto. The resultant mixture was thoroughly mixed and, then, the remaining hydroxypropyl cellulose were added and thoroughly mixed in a mixer. Thereafter, 0.5 part of ascorbic acid, which is different from unsaturated lower aliphatic monocarboxylic acid having six or less carbon atoms used in the present invention, was added and thoroughly mixed.

Thus, a powdery composition for the application to the oral cavity containing beclomethasone dipropionate uniformly dispersed therein was prepared. The powdery composition was filled in #2 hard gelatin capsules in an amount of 200 mg each to prepare a powdery preparation in a unit dosage form for the application to the oral cavity.

The capsules thus prepared were allowed to stand at a temperature of 60°C for 30 days. The residual percentage of beclomethasone dipropionate in the capsules were determined.

The results were as shown in Table 13.

TABLE 13

| Storage period (day) | 7 | 14 | 21 | 30 |
|---|---|---|---|---|
| Residual % of beclo-methasone dipropionate | 94.5 | 89.5 | 82.0 | 79.0 |

Comparative Example 2

A powdery preparation in a unit dosage form for the application to the oral cavity was prepared in the same manner as in Comparative Example 1, except that glutamic acid, which is different from aliphatic polycarboxylic acid used in the present invention, was used instead of the ascorbic acid.

The capsules obtained above were allowed to stand at a temperature of 60°C for 30 days. The residual percentages of the beclomethasone dipropionate in the capsules were determined.

The results were as shown in Table 14.

TABLE 14

| Storage period (day) | 7 | 14 | 21 | 30 |
|---|---|---|---|---|
| Residual % of beclo-methasone dipropionate | 95.5 | 91.2 | 88.5 | 83.2 |

Comparative Example 3

A powdery preparation in a unit dosage form for the application to the oral cavity was prepared in the same manner as in Comparative Example 1, except that sodium carboxymethyl cellulose having carboxylate groups different from carboxyl groups and used instead of the ascorbic acid.

The capsules obtained above were allowed to stand at a temperature of 60°C for 30 days. The residual percentages of the beclomethasone dipropionate in the capsules were determined.

The results were as shown in Table 15.

TABLE 15

| Storage period (day) | 7 | 14 | 21 | 30 |
|---|---|---|---|---|
| Residual % of beclo-methasone dipropionate | 93.0 | 86.2 | 80.0 | 75.1 |

Comparative Example 4

A powdery preparation in a unit dosage form for the application to the oral cavity was prepared in the same manner as in Comparative Example 1, except that aspirin, which is different from steroid or glycyrrhizic acid type anti-inflammatory agents used in the present invention, was used instead of beclomethasone dipropionate and stearic acid, which is used as a stabilizer in the present invention, was used instead of the ascorbic acid.

The capsules obtained above were allowed to stand at a temperature of 60°C for 30 days. The residual percentages of the aspirin in the capsules were determined.

The results were as shown in Table 16.

TABLE 16

| Storage period (day) | 7 | 14 | 21 | 30 |
|---|---|---|---|---|
| Residual % of aspirin | 92.2 | 85.5 | 78.2 | 72.0 |

Comparative Example 5

A powdery preparation in a unit dosage form for the application to the oral cavity was prepared in the same manner as in Comparative Example 1, except that azulen, which is different from steroid or glycyrrhizic acid type anti-inflammatory agents used in the present invention, was used instead of the beclomethasone dipropionate and palmitic acid, which is used as a stabilizer in the present invention, was used instead of the ascorbic acid.

The capsules obtained above were allowed to stand at a temperature of 60°C for 30 days. The residual percentages of the azulen in the capsules were determined.

The results were as shown in Table 17.

TABLE 17

| Storage period (day) | 7 | 14 | 21 | 30 |
|---|---|---|---|---|
| Residual % of azulen | 92.0 | 81.5 | 74.2 | 65.5 |

Comparative Example 6

A powdery preparation in a unit dosage form for the application to the oral cavity was prepared in the same manner as in Comparative Example 1, except that magnesium stearate, which is different from stearic acid used in the present invention, was used instead of the ascorbic acid.

The capsules obtained above were allowed to stand at a temperature of 60°C for 30 days. The residual percentages of the beclomethasone dipropionate in the capsules were determined.

The results were as shown in Table 18.

TABLE 18

| Storage period (day) | 7 | 14 | 21 | 30 |
|---|---|---|---|---|
| Residual % of beclo-methasone dipropionate | 94.3 | 90.5 | 85.4 | 80.4 |

Comparative Example 7

A 100 part amount of hydroxypropyl cellulose having a viscosity of a 2% aqueous solution thereof of 1550 mPas (cps) and having a particle size distribution such that 90% or more of the powder particles had a particle diameter of 37 to 149 µm was used.

A 10 part amount of the hydroxypropyl cellulose and 0.17 part of beclomethasone dipropionate were thoroughly mixed in a mixer and, then, the remaining hydroxypropyl cellulose was added thereto, followed by mixing. Thereafter, 0.5 part of ascorbic acid was added and thoroughly mixed. Thus, a powdery pharmaceutical composition for the application to the nasal cavity containing beclomethasone dipropionate uniformly dispersed therein was prepared.

The powdery composition obtained above was filled in #2 hard gelatin capsules in an amount of 30 mg each to prepare a powdery preparation in a unit dosage form for the application to the nasal cavity.

The residual percentages of the beclomethasone dipropionate were determined by allowing the capsules to stand at a temperature of 60°C for 30 days.

The results were as shown in Table 19.

TABLE 19

| Storage period (day) | 7 | 14 | 21 | 30 |
|---|---|---|---|---|
| Residual % of beclo-methasone dipropionate | 93.6 | 90.1 | 82.2 | 79.1 |

Comparative Example 8

A powdery preparation in a unit dosage form for the application to the nasal cavity was prepared in the same manner as in Comparative Example 7, except that glutamic acid was used instead of the ascorbic acid.

The capsules obtained above were allowed to stand at a temperature of 60°C for 30 days. The residual percentages of the beclomethasone dipropionate in the capsules were determined.

The results were as shown in Table 20.

TABLE 20

| Storage period (day) | 7 | 14 | 21 | 30 |
|---|---|---|---|---|
| Residual % of beclo-methasone dipropionate | 93.5 | 89.2 | 85.5 | 80.2 |

Comparative Example 9

A powdery preparation in a unit dosage form for the application to the nasal cavity was prepared in the same manner as in Comparative Example 7, except that aspirin was used instead of beclomethasone dipropionate and stearic acid was used instead of the ascorbic acid.

The capsules obtained above were allowed to stand at a temperature of 60°C for 30 days. The residual percentages of the aspirin in the capsules were determined.

The results were as shown in Table 21.

TABLE 21

| Storage period (day) | 7 | 14 | 21 | 30 |
|---|---|---|---|---|
| Residual % of aspirin | 94.2 | 87.2 | 80.5 | 75.1 |

Comparative Example 10

A powdery preparation in a unit dosage form for the application to the oral cavity was prepared in the same manner as in Comparative Example 7, except that magnesium stearate was used instead of the ascorbic acid.

The capsules obtained above were allowed to stand at a temperature of 60°C for 30 days. The residual percentages of the beclomethasone dipropionate in the capsules were determined.

The results were as shown in Table 14.

TABLE 22

| Storage period (day) | 7 | 14 | 21 | 30 |
|---|---|---|---|---|
| Residual % of beclo-methasone dipropionate | 95.3 | 91.5 | 86.4 | 81.5 |

Example 21

A 100 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 µm was used.

A 10 part amount part of the hydroxypropyl cellulose and 0.17 part of beclomethasone dipropionate were thoroughly mixed in a mixer and, then, the remaining hydroxypropyl cellulose was gradually added thereto. The resultant mixture was thoroughly mixed. Thereafter, 0.5 part of stearic acid was added and thoroughly mixed.

Thus, a powdery composition for the application to the nasal cavity containing beclomethasone dipropionate uniformly dispersed therein was prepared. The powdery composition was filled in #2 hard gelatin capsules in an amount of 30 mg each to prepare a powdery preparation in a unit dosage form for the application to the nasal cavity.

The capsules thus prepared were allowed to stand at a temperature of 60°C for 30 days or at 40°C for 6 month. The residual percentages of beclomethasone dipropionate in the capsules were determined. The results were as shown in Table 23.

TABLE 23

| 60°C | Storage period (day) | 7 | 14 | 21 | 30 |
|---|---|---|---|---|---|
| | Residual % of beclo-methasone dipropionate | 99.0 | 98.7 | 98.0 | 96.7 |
| 40°C | Storage period (month) | 1 | 2 | 3 | 6 |
| | Residual % of beclo-methasone dipropionate | 99.9 | 99.5 | 99.0 | 98.0 |

Comparative Example 11

A powdery preparation for the application to the oral cavity in the same manner as in Example 1, except that capric acid (i.e., a saturated higher aliphatic monocarboxylic acid having 10 carbon atoms) was used instead of the stearic acid.

However, the powdery preparation obtained above was not appropriate as a powdery preparation because, when the powdery preparation filled in the capsule was administered to the oral cavity by the above-explained spraying device, the odor was too strong.

Example 22

A 100 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 μm was used.

A 10 part amount part of the hydroxypropyl cellulose and 1.7 parts of triaminolone acetonide were thoroughly mixed in a mixer and, then, the remaining hydroxypropyl cellulose was gradually added thereto. The resultant mixture was thoroughly mixed. Thereafter, 0.5 part of stearic acid and 0.3 part of magnesium stearate were added and thoroughly mixed. Thus, a powdery composition for the application to the nasal cavity containing triamcinolone acetonide uniformly dispersed therein was prepared.

The powdery composition obtained above was pulverized by a coarse grinding machine and, then, compressed to prepare tablets having a Monsanto hardness of 3 to 4 kg. The tablets were pulverized by the grinding machine to prepare the powder particles. The powder particles thus obtained were sieved to obtain the powder particles having a particle size of 60 to 400 meshes. The powder particles were, then, filled in #2 hard gelatin capsules in an amount of 30 mg each to prepare a powdery preparation in a unit dosage form for the application to the nasal cavity.

Example 23

A 100 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 μm was charged into a fluidized bed type granulator. Then, 50 parts of, ethanol mixture containing 0.025 part of beclomethasone dipropionate and 5 parts of hydroxypropyl cellulose having a viscosity of a 2% aqueous solution thereof of 7 mPas (cps) at 37°C±0.2°C dissolved therein and also containing 0.5 part of stearic acid dissolved therein were sprayed from the spraying nozzle of the granulator, followed by drying. Thus, the powder particles impregnated with the solid components in the ethanol mixture were obtained. The powder particles were sieved to obtain those having a particle size of 60 to 400 meshes. The powder particles thus obtained were filled in #2 hard gelatin capsules in an amount of 200 mg each to prepare a powdery preparation in a unit dosage form for the application to the oral cavity.

Example 24

A 80 part amount of hydroxypropyl cellulose having a viscosity of a 2% aqueous solution thereof of 1550 mPas (cps) at 37°C±0.2°C and having a particle size such that 90% or more of the powder particles had a particle diameter of 37 to 149 μm and 1000 parts of an ethanol suspension containing 0.2 part of the dissolved beclomethasone dipropionate and 0.4 part of the suspended stearic acid were casted on the surface of releasing paper. After drying, the casted film was obtained.

The film obtained above was ground and pulverized by a grinding machine and, then, sieved to the powder particles having a particle size of 60 to 400 meshes. The powder particles were filled in #2 hard gelatin capsules in an amount of 200 mg each to prepare a powdery preparation in a unit dosage form for the application to the oral cavity.

# EP 0 187 433 B1

**Claims**

1. A powdery pharmaceutical composition for application to the mucosa of the oral or nasal cavity comprising a base selected from lower alkyl ethers of cellulose, a pharmaceutically effective amount of a drug selected from steroidal anti-inflammatory agents or glycyrrhizic acid anti-inflammatory agents and, as a solid stabilizer for the drug, at least one low irritant solid organic acid selected from saturated higher aliphatic monocarboxylic acids having 12 to 24 carbon atoms, aliphatic hydrocarbon dicarboxylic acids having 4 to 6 carbon atoms, hydroxy aliphatic hydrocarbon di-carboxylic and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition.

2. A powdery pharmaceutical composition as claimed in Claim 1, wherein said stabilizer is contained in an amount of 0.1% to 3% by weight in the composition.

3. A powdery pharmaceutical composition as claimed in Claim 1 or 2, wherein at least about 90% by weight of the stabilizer particles has an effective particle diameter of about 75 µm or less.

4. A powdery pharmaceutical composition as claimed in Claim 1, for application to the mucosa of the oral cavity.

5. A powdery pharmaceutical composition as claimed in any of Claims 1 to 4, wherein said stabiliser is stearic acid, palmitic acid, lauric acid, or myristic acid.

6. A powdery pharmaceutical composition as claimed in any of Claims 1 to 4, wherein said stabiliser is succinic acid, fumaric acid, or maleic acid.

7. A powdery pharmaceutical composition as claimed in any of Claims 1 to 4, wherein said stabiliser is tartaric acid or citric acid.

8. A powdery pharmaceutical composition as claimed in any of Claims 1 to 4, wherein said aromatic carboxylic acid is benzoic acid, parahydroxybenzoic acid, or salicylic acid.

9. A powdery pharmaceutical composition as claimed in any of the preceding claims wherein said steroid anti-inflammatory agent is beclomethasone dipropionate, betamethasone, betamethasone valerate, trimcinolone, triamcinolone acetonide, dexamethasone, fluocinolone acetonide, fluocinonide, flumethasone, hydrocortisone, prednisolone, or prednisone.

10. A powdery pharmaceutical composition as claimed in any of Claims 1 to 8, wherein said glycyrrhizic acid type anti-inflammatory agent is glycyrrhizic acid, disodium glycyrrhizinate trisodium glycyrrhizinate, monopotassium glycyrrhizinate, dipotassium glycyrrhizinate or monoammonium glycyrrhizinate.

11. A powdery pharmaceutical composition as claimed in Claim 1, wherein the moisture content of the lower alkyl ether of cellulose is within the range of from 3% to 9% by weight.

12. A powdery pharmaceutical composition as claimed in any of the preceding claims, wherein said lower alkyl ether of cellulose is hydroxypropyl cellulose, methyl cellulose, or hydroxypropyl methyl cellulose.

13. A powdery pharmaceutical composition as claimed in any of the preceding claims, wherein at least about 90% by weight of the powder particles of the pharmaceutical composition has an effective particle diameter of about 20 to 250 µm.

14. A powdery pharmaceutical preparation in unit dosage form for application to the mucosa of the oral or nasal cavity comprising the powdery pharmaceutical composition of Claim 1.

15. A powdery pharmaceutical preparation as claimed in Claim 14, wherein said pharmaceutical composition is filled in a capsule.

16. A method for preparing a powdery pharmaceutical composition for application to the mucosa of the oral or nasal cavity comprising the steps of mechanically mixing
(a) a base selected from lower alkyl ethers of cellulose;
(b) a pharmaceutically effective amount of a drug selected from steroidal anti-inflammatory agents or glycyrrhizic acid anti-inflammatory agents; and
(c) a solid stabilizer for the drug composed of at least one low irritant solid organic acid selected from saturated higher aliphatic monocarboxylic acids having 12 to 24 carbon atoms, aliphatic hydrocarbon dicarboxylic acids having 4 to 6 carbon atoms, hydroxy aliphatic hydrocarbon di- and tricarboxylic acids having 4 to 8 carbon atoms, aromtic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition.

17. A method as claimed in Claim 16, wherein the mixed composition is compressed under pressure and the compressed mixture is then pulverized.

18. A method for preparing a powdery pharmaceutical composition for application to the mucosa of the oral or nasal cavity comprising the steps of impregnating
a base selected from lower alkyl ethers of cellulose with a solution or dispersion, in an organic solvent, of
a pharmaceutically effective amount of a drug selected from steroidal anti-inflammatory agents or glycyrrhizic acid anti-inflammatory agents; and
a solid stabilizer for the drug composed of at least one low irritant solid organic acid selected from

14

saturated higher aliphatic monocarboxylic acids having 12 to 24 carbon atoms, aliphatic hydrocarbon dicarboxylic acids having 4 to 6 carbon atoms, hydroxy aliphatic hydrocarbon di- and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition, and drying the impregnated product.

19. A method for preparing a powdery pharmaceutical composition for application to the mucosa of the oral or nasal cavity comprising the steps of dissolving or dispersing, in an organic solvent, water or the mixture thereof

(a) a base selected from lower alkyl ethers of cellulose;

(b) a pharmaceutically effective amount of a drug selected from steroidal anti-inflammatory agents or glycyrrhizic acid anti-inflammatory agents; and

(c) a solid stabilizer for the drug composed of at least one low irritant solid organic acid selected from saturated higher aliphatic monocarboxylic acids having 12 to 24 carbon atoms, aliphatic hydrocarbon dicarboxylic acids having 4 to 6 carbon atoms, hydroxy aliphatic hydrocarbon di- and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition;

evaporating the organic solvent, water, or the mixture thereof from the resultant solution or dispersion; and

pulverising the evaporated product.

20. A method for stabilising a powdery pharmaceutical composition containing a drug selected from steroidal anti-inflammatory agents or glycyrrhizic acid anti-inflammatory agents for application to the mucosa of the oral or nasal cavity comprising incorporating a solid stabilizer composed of at least one low irritant solid organic acid selected from saturated higher aliphatic monocarboxylic acids having 12 to 24 carbon atoms, aliphatic hydrocarbon dicarboxylic acids having 4 to 6 carbon atoms, hydroxy aliphatic hydrocarbon di- and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition.

21. In the preparation of a powdery pharmaceutical composition for application to the mucosa of the oral or nasal cavity comprising a base selected from lower alkyl ethers of cellulose and a pharmaceutically effective amount of a drug selected from steroidal anti-inflammatory agents or glycyrrhizic acid anti-inflammatory agents, the use, as a solid stabilizer for the drug, of at least one low irritant solid organic acid selected from saturated higher aliphatic monocarboxylic acids having 12 to 24 carbon atoms, aliphatic hydrocarbon dicarboxylic acids having 4 to 6 carbon atoms, hydroxy aliphatic hydrocarbon di- and tricarboxylic acids having 4 to 8 carbon atoms, aromatic carboxylic acids, unsaturated lower aliphatic monocarboxylic acids having six or less carbon atoms, and carboxymethyl cellulose and carboxymethylethyl cellulose, said stabilizer being present in an amount of 0.05% to 5% by weight in the composition.

**Patentansprüche**

1. Pulverige pharmazeutische Zusammensetzung zur Anwendung auf die Schleimhaut der Mund- oder Nasenhöhle, umfassend eine Basis, die ausgewählt ist unter Zelluloseäthern niederer Alkyhle, eine pharmazeutisch wirksame Menge einer Droge (eines Arzneimittels), die ausgewählt ist unter steroiden Anti-Entzündungsmittels oder Glycyrrhizinsäure- Anti-Entzündungsmitteln und als festen Stabilisator für die Droge mindestens eine wenig irritierende, feste organische Säure, die ausgewählt ist unter gesättigten Monocarboxylsäuren höhere Aliphate mit 12 bis 24 Kohlenstoffatomen, aliphatischen Kohlenwasserstoffdicarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigen Monocarboxylsäuren niedere Aliphate mit 6 oder weniger Kohlenstoffatomen und Carboxymethylzellulose sowie Carboxymethyläthylzellulose, wobei der Stabilisator in der Zusammensetzung in einer Menge von 0,05 bis 5 Gew.-% vorhanden ist.

2. Pulverige pharmazeutische Zusammensetzung nach Anspruch 1, in der der Stabilisator in der Zusammensetzung in einer Menge von 0,1 bis 3 Gew.-% enthalten ist.

3. Pulverige pharmazeutische Zusammensetzung nach Anspruch 1 oder 2, in der mindestens etwa 90 Gew.-% der Stabilisatorpartikel einen effektiven Partikeldurchmesser von etwa 75 m oder weniger besitzen.

4. Pulverige pharmazeutische Zusammensetzung nach Anspruch 1 zur Anwendung auf die Schleimhaut der Mundhöhle.

5. Pulverige pharmazeutische Zusammensetzung nach einem der Ansprüche 1 bis 4, bei der der Stabilisator Stearinsäure, Palmitinsäure, Laurinsäure oder Myristinsäure ist.

6. Pulverige pharmazeutische Zusammensetzung nach einem der Ansprüche 1 bis 4, bei der der Stabilisator Sukzinsäure, Fumarsäure oder Maleinsäure ist.

7. Pulverige pharmazeutische Zusammensetzung nach einem der Ansprüche 1 bis 4, bei der der Stabilisator Tartarsäure oder Zitronensäure ist.

8. Pulverige pharmazeutische Zusammensetzung nach einem der Ansprüche 1 bis 4, bei der die aromatische Carboxylsäure Benzoesäure, Parahydroxybenzoesäure oder Salicylsäure ist.

9. Pulverige pharmazeutische Zusammensetzung nach irgend einem der vorangegangenen Ansprüche, bei der das steroide Anti-Entzündungsmittel Beciomethasondipropionat, Betamethason, Betamethasolvalerat, Triamcinolon, Triamcinolonacetonid, Dexamethason, Fluocinolonacetonid, Fluocinonid, Flumethason, Hydrocortison, Prednisolon oder Prednison ist.

10. Pulverige pharmazeutische Zusammensetzung nach einem der Ansprüche 1 bis 8, bei der das Anti-Entzündungsmittel vom Glycyrrhzinsäuretyp Glycyrrhzinsäure, Dinatriumglycyrrhizinat, Trinatriumglycyrrhizinat, Monokaliumglycyrrhizinat, Dikaliumglycyrrhizinat oder Monoammoniumglycyrrhizinat ist.

11. Pulverige pharmaceutische Zusammensetzung nach Anspruch 1, bei der der Feuchtigkeitsgehalt des niederalkylen Celluloseäthers in dem Bereich von 3 bis 9 Gew.-% liegt.

12. Pulverige pharmazeutische Zusammensetzung nach irgend einem der vorangegangenen Ansprüche, bei der der niederalkyle Celluloseäther Hydroxypropylzellulose, Methylzellulose oder Hydroxypropylmethylzellulose ist.

13. Pulverige pharmazeutische Zusammensetzung nach irgend einem der vorangegangenen Ansprüche, bei der mindestens etwa 90 Gew.-% der Pulverpartikel der pharmazeutischen Zusammensetzung einen effektiven Partikeldurchmesser von etwa 20 bis 250 m besitzen.

14. Pulveriges pharmazeutisches Präparat in Form einer Einheitsdosis zur Anwendung auf die Schleimhaut der Mund- oder Nasenhöhle, welche die pulverige pharmazeutische Zusammensetzung gemäß Anspruch 1 umfaßt.

15. Pulveriges pharmazeutisches Präparat nach Anspruch 14, bei dem die pharmazeutische Zusammensetzung in eine Kapsel gefüllt ist.

16. Verfahren zum Herstellen einer pulverigen pharmazeutischen Zusammensetzung zur Anwendung auf die Schleimhaut der Mund- oder Nasenhöhle, welches die (folgenden) Schritte umfaßt:
es werden mechanisch gemischt:
a) eine Basis, die ausgewählt ist unter niederalkylen Zeulloseäthern;
b) eine pharmazeutische wirksame Menge einer Droge (eines Arzneimittels), welches ausgewählt ist unter steroiden Anti-Entzündungsmitteln oder Glycyrrhizinsäure-Anti-Entzündungsmitteln; und
c) ein fester Stabilisator für die Droge, welcher zusammengesetzt ist aus mindestens einer wenig irritierenden festen, organischen Säure, die ausgewählt ist unter gesättigten Monocarboxylsäuren höhere Aliphate mit 12 bis 24 Kohlenstoffatomen, aliphatischen Kohlenwasserstoffdicarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigten Monocarboxylsäuren niederer Aliphate mit 6 oder weniger Kohlenstoffatomen und Carboxymethylzellulose sowie Carboxymethyläthylzellulose, wobei der Stabilisator in der Zusammensetzung in einer menge von 0,05 bis 5 Gew.-% vorhanden ist.

17. Verfahren nach Anspruch 16, bei dem die gemischte Zusammensetzung unter Druck komprimiert wird und bei dem die komprimierte Mischung dann pulverisiert wird.

18. Verfahren zum Herstellen einer pulverigen pharmazeutischen Zusammensetzung zur Anwendung auf die Schleimhaut der Mund- oder Nasenhöhle, welche die (folgenden) Schritte umfaßt:
man imprägniert:
eine Basis, welche ausgewählt ist unter niederalkylen Zelluloseäthern mit einer Lösung oder Dispersion in einem organischen Lösungsmittel, von einer pharmazeutisch wirksamen Menge einer Droge (eines Arzneimittels), die ausgewählt ist unter steroiden Anti-Entzündungsmitteln oder
Glycyrrhizinsäure-Anti-Entzündungsmitteln; und
eines festen Stabilisators für die Droge, der zusammengesetzt ist aus mindestens einer wenig irritierenden, feste organische Säure, die ausgewählt ist unter gesättigten Monocarboxylsäuren höherer Aliphate mit 12 bis 24 Kohlenstoffatomen, aliphatischen Kohlenwasserstoffdcarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigten Monocarboxylsäuren niederer Aliphate mit 6 oder weniger Kohlenstoffatomen und Carboxymethylzellulose sowie Carboxymethyläthylzellulose, wobei der Stabilisator in der Zusammensetzung in einer Menge von 0,05 bis 5 Gew.-% vorhanden ist, und man trocknet das imprägniert Produkt.

19. Verfahren zum Herstellen einer pulverigen pharmazeutischen Zusammensetzung zur Anwendung auf die Schleimhaut der Mund- oder Nasenhöhle, welches die (folgenden) Schritte umfaßt:
in einem organischen Lösungsmittel, in Wasser oder einer Mischung derselben (dieser Stoffe) werden gelöst oder dispergiert:
a) eine Basis, die ausgewählt ist unter den niederalkylen Zelluloseäthern;
b) eine pharmazeutisch wirksame Menge einer Droge (eines Arzneimittels), welches ausgewählt ist unter steroiden Anti-Entzündungsmitteln oder Glycyrrhizinsäure-Anti-Entzündungsmitteln; und
c) einen festen Stabilisator für die Droge, welche zusammengesetzt ist aus mindestens einer wenig irritierenden, festen, organischen Säure, die ausgewählt ist unter gesättigten Monocarboxylsäuren höherer

# EP 0 187 433 B1

Aliphate mit 12 bis 24 Kohlenstoffatomen, aliphatischen Kohlenwasserstoffdicarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigten Monocarboxylsäuren niederer Aliphate mit 6 oder weniger Kohlenstoffatomen und Carboxymethylzellulose sowie Carboxymethyläthylzellulose, wobei der Stabilisator in der Zusammensetzung in einer Menge von 0,05 bis 5 Gew.-% vorhanden ist;

man verdampft das organische Lösungsmittel, Wasser oder die Mischung derselben aus der resultierenden Lösung oder Dispersion; und

man pulverisiert das beim Verdampfen erhaltene Produkt.

20. Verfahren zum Stabilisieren einer pulverigen pharmazeutischen Zusammensetzung, welche eine Droge (ein Arzneimittel) enthält, welche ausgewählt ist unter steroiden Anti-Entzündungsmitteln oder Glycyrrhizinsäure-Antientzündungsmitteln, zur Anwendung auf die Schleimhaut der Mund- oder Nasenhöhle, umfassend das Einbringen eines festen Stabilisators, der zusammengesetzt ist aus mindestens einer wenig irritierenden, festen organischen Säure, die ausgewählt ist unter gesättigten Monocarboxylsäuren höherer Aliphate mit 12 bis 24 Kohlenstoffatomen, aliphatischen Kohlenwasserstoffdicarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigten Monocarboxylsäuren niederer Aliphate mit 6 oder weniger Kohlenstoffatomen und Carboxymethylzellulose sowie Carboxymethyläthylzellulose, wobei der Stabilisator in der Zusammensetzung in einer Menge von 0,05 bis 5 Gew.-% vorhanden ist.

21. Bei der Herstellung einer pulverigen pharmazeutischen Zusammensetzung zur Anwendung auf die Schleimhaut der Mund- oder Nasenhöhle, welche (Zusammensetzung) eine Basis umfaßt, die unter den niederalkylen Zelluloseäthern ausgewählt ist, sowie eine pharmazeutisch wirksame Menge einer Droge, die ausgewählt ist unter den steroiden Anti-Entzündungsmitteln oder den Glycyrrhizinsäure-Anti-Entzündungsmitteln, die Verwendung—als fester Stabilisator für die Droge—mindestens einer wenig irritierenden, festen organischen Säure, die ausgewälhlt ist unter gesättigten Monocarboxylsäuren höherer Aliphate mit 12 bis 24 Kohlenstoffatomen, aliphatischen Kohlenwasserstoffdicarboxylsäuren mit 4 bis 6 Kohlenstoffatomen, hydroxyaliphatischen Kohlenwasserstoffdicarboxyl- und -Tricarboxylsäuren mit 4 bis 8 Kohlenstoffatomen, aromatischen Carboxylsäuren, ungesättigten Monocarboxylsäuren niederer Aliphate mit 6 oder weniger Kohlenstoffatomen und Carboxymethylzellulose sowie Carboxymethyläthylzellulose, wobei der Stabilisator in der Zusammensetzung in einer Menge von 0,05 bis 5 Gew.-% vorhanden ist.

**Revendications**

1. Composition pharmaceutique poudreuse pour application à la muqueuse de la cavité orale ou nasale, comprenant une base choisie parmi les éthers de cellulose à alcoyle inférieur, une quantité pharmaceutiquement efficace d'un médicament choisi parmi les agents anti-inflammatoires stéroïdiques ou les agents anti-inflammatoires à base d'acide glycyrrhizique, et

comme stabilisateur solide pour le médicament, au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides dicarboxyliques et tricarboxyliques hydrocarbonés hydroxyaliphatiques ayant de 4 à 8 atomes de carbone, les acides carboxyliques aromatiques, les acides monocarboxyliques aliphatiques inférieurs non-saturés ayant 6 atomes de carbone ou moins, la carboxyméthylcellulose et la carboxyméthyléthylcellulose, ledit stabilisateur étant présent en une quantité de 0,05% à 5% en poids de la composition.

2. Composition pharmaceutique poudreuse selon la revendication 1, où ledit stabilisateur est contenu en une quantité de 0,1% à 3% en poids de la composition.

3. Composition pharmaceutique poudreuse selon la revendication 1 ou 2, où au moins environ 90% en poids des particules de stabilisateur ont un diamètre particulaire effectif d'environ 75 µm ou moins.

4. Composition pharmaceutique poudreuse selon la revendication 1 pour application à la muqueuse de la cavité orale.

5. Composition pharmaceutique poudreuse selon l'une quelconque des revendications 1 à 4, où ledit stabilisateur est l'acide stéarique, l'acide palmitique, l'acide laurique ou l'acide myristique.

6. Composition pharmaceutique poudreuse selon l'une quelconque des revendications 1 à 4, où ledit stabilisateur est l'acide succinique, l'acide fumarique, ou l'acide maléique.

7. Composition pharmaceutique poudreuse selon l'une quelconque des revendications 1 à 4, où ledit stabilisateur est l'acide tartrique ou l'acide citrique.

8. Composition pharmaceutique poudreuse selon l'une quelconque des revendications 1 à 4, où ledit acide carboxylique aromatique est l'acide benzoïque, l'acide parahydroxybenzoïque ou l'acide salicylique.

9. Composition pharmaceutique poudreuse selon l'une quelconque des revendications précédentes, où ledit agent anti-inflammatoire stéroïdique est le dipropionate de béclométhasone, la bêtaméthasone, le valérate de bêtaméthasone, la triamcinolone, l'acétonide de triamcinolone, la dexaméthasone, l'acétonide de fluocinolone, le fluocinonide, la fluméthasone, l'hydrocortisone, la prednisolone, ou la prednisone.

10. Composition pharmaceutique poudreuse selon l'une quelconque des revendications 1 à 8, où ledit

17

agent anti-inflammatoire de type acide glycyrrhizique est l'acide glycyrrhizique, le glycyrrhizinate disodique, le glycyrrhizinate trisodique, le glycyrrhizinate monopotassique, le glycyrrhizinate dipotassique ou le monoglycyrrhizinate d'ammonium.

11. Composition pharmaceutique poudreuse selon la revendication 1, où la teneur en humidité de l'éther de cellulose à alcoyle inférieur est compris dans l'intervalle allant de 3% à 9% en poids.

12. Composition pharmaceutique poudreuse selon l'une quelconque des revendications précédentes, où ledit éther de cellulose à alcoyle inférieur est l'hydroxypropylcellulose, la méthylcellulose ou l'hydroxypropylméthylcellulose.

13. Composition pharmaceutique poudreuse selon l'une quelconque des revendications précédentes, où au moins environ 90% en poids des particules de poudre de la composition pharmaceutique ont un diamètre particulaire effectif d'environ 20 à 250 μm.

14. Préparation pharmaceutique poudreuse sous forme de dose unitaire pour application à la cavité orale ou nasale, comprenant la composition pharmaceutique poudreuse de la revendication 1.

15. Préparation pharmaceutique poudreuse selon la revendication 14, où ladite composition pharmaceutique est versée dans une capsule.

16. Procédé pour préparer une composition pharmaceutique poudreuse pour application à la muqueuse de la cavité orale ou nasale, comprenant les étapes de mélange mécanique de

(a) un base choisie parmi les éthers de cellulose à alcoyle inférieur;

(b) une quantité pharmaceutiquement efficace d'un médicament choisi parmi les agents anti-inflammatoires stéroïdiques ou les agents anti-inflammatoires à base d'acide glycyrrhizique; et

(c) un stabilisateur seolide pour le médicament, composé d'au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides di- et tricarboxyliques hydrocarbonés hydroxyaliphatiques ayant de 4 à 8 atomes de carbone, les acides carboxyliques aromatiques, les acides monocarboxyliques aliphatiques inférieurs non-saturés ayant 6 atomes de carbone ou moins, la carboxyméthylcellulose et la carboxyméthyléthylcellulose, ledit stabilisateur étant présent en une quantité de 0,05% à 5% en poids de la composition.

17. Procédé selon la revendication 16, où la composition mélangée est comprimée sous pression et le mélange comprimé est alors pulvérisé.

18. Procédé de préparation d'une composition pharmaceutique poudreuse pour application à la muqueuse de la cavité orale ou nasale, comprenant les étapes qui consistent à imprégner

une base choisie parmi les éthers de cellulose à alcoyle inférieur, avec une solution ou dispersion, dans un solvant organique, de

une quantité pharmaceutiquement efficace d'un médicament choisi parmi les agents anti-inflammatoires stéroïdiques ou les agents anti-inflammatoires à base d'acide glycyrrhizique; et

un stabilisateur solide pour le médicament, composé d'au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides di- et tricarboxyliques hydrocarbonés hydroxyaliphatiques ayant de 4 à 8 atomes de carbone, les acides carboxyliques aromatiques, les acides monocarboxyliques aliphatiques inférieurs non-saturés ayant 6 atomes de carbone ou moins, la carboxyméthylcellulose et la carboxyméthyléthylcellulose, ledit stabilisateur étant présent en une quantité de 0,05% à 5% en poids dans la composition, et à sécher le produit imprégné.

19. Procédé de préparation d'une composition pharmaceutique poudreuse pour application à la muqueuse de la cavité orale ou nasale, comprenant les étapes qui consistent à dissoudre ou disperser, dans un solvant organique, l'eau ou leur mélange,

(a) une base choisie parmi les éthers de cellulose à alcoyle inférieur,

(b) une quantité pharmaceutique efficace d'un médicament choisi parmi les agents anti-inflammatoires stéroïdiques ou les agents anti-inflammatoires à base d'acide glycyrrhizique; et

(c) un stabilisateur solide pour le médicament, composé d'au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides di- et tricarboxyliques hydrocarbonés hydroxyaliphatiques ayant de 4 à 8 atomes de carbone, les acides carboxyliques aromatiques, les acides monocarboxyliques aliphatiques inférieurs non-saturés ayant 6 atomes de carbone ou moins, la carboxyméthylcellulose et la carboxyméthyléthylcellulose, ledit stabilisateur étant présent en une quantité de 0,05% à 5% en poids de la composition,

à évaporer le solvant organique, l'eau ou leur mélange de la solution ou dispersion résultante; et

à pulvériser le produit évaporé.

20. Procédé pour stabiliser une composition pharmaceutique poudreuse contenant un médicament choisi parmi les agents anti-inflammatoires stéroïdiques ou les agents anti-inflammatoires à base d'acide glycyrrhizique pour application à la muqueuse de la cavité oral ou nasale, dans lequel on incorpore un stabilisateur solide composé d'au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides di- et tricarboxyliques hydrocarbonés hydroxyaliphatiques ayant de 4 à 8 atomes de carbone, les acides

18

carboxyliques aromatiques, les acides monocarboxyliques aliphatiques inférieurs non-saturés ayant 6 atomes de carbon ou moins, la carboxyméthyl-cellulose et la carboxyméthyléthylcellulose, ledit stabilisateur étant présent en une quantité de 0,05% à 5% en poids de la composition.

21. Dans la préparation d'une composition pharmaceutique poudreuse pour application à la muqueuse de la cavité orale ou nasale, comprenant une base choisie parmi les éthers de cellulose à alcoyle inférieur et une quantité pharmaceutiquement efficace d'un médicament choisi parmi les agents anti-inflammatoires stéroïdiques ou les agents anti-inflammatoires à base d'acide glycyrrhizique, l'utilisation, comme stabilisateur solide pour le médicament, d'au moins un acide organique solide faiblement irritant choisi parmi les acides monocarboxyliques aliphatiques supérieurs saturés ayant de 12 à 24 atomes de carbone, les acides dicarboxyliques hydrocarbonés aliphatiques ayant de 4 à 6 atomes de carbone, les acides di- et tricarboxyliques hydrocarbonés hydroxyaliphatiques ayant de 4 à 8 atomes de carbone, les acides carboxyliques aromatiques, les acides monocarboxyliques aliphatiques inférieurs non-saturés ayant 6 atomes de carbone ou moins, la carboxyméthylcellulose et la carboxyméthyléthylcellulose, ledit stabilisateur étant présent en une quantité de 0,05% à 5% en poids de la composition.